# EUROPÄISCHE PATENTSCHRIFT

(11) **EP 0 782 585 B1**
(45) Veröffentlichungstag und Bekanntmachung des Hinweises auf die Patenterteilung: **14.06.2000**
(21) Anmeldenummer: 95937047.9
(22) Anmeldetag: 02.11.1995
(51) Int. Cl.: C07K 16/00, C07K 16/06, G01N 33/563, G01N 33/68, G01N 33/543

(54) **RHEUMAFAKTOR-ENTSTÖRUNG MIT ANTI-Fd-ANTIKÖRPERN**
ELIMINATION OF RHEUMATOID FACTOR INTERFERENCE USING ANTI-Fd ANTIBODIES
ELIMINATION D'INTERFERENCES DUES AU FACTEUR RHUMATOIDE AU MOYEN D'ANTICORPS ANTI-Fd

(30) Priorität: 04.11.1994 DE 4439452
(43) Veröffentlichungstag der Anmeldung: 09.07.1997
(73) Patentinhaber: Roche Diagnostics GmbH, 68305 Mannheim (DE)
(72) Erfinder: KLEMT, Volker, 82362 Weilheim (DE); SCHLIEPER, Dittmar, 82393 Iffeldorf (DE); SCHMITT, Urban, 82386 Oberhausen (DE); WIEDMANN, Michael, 82377 Penzberg (DE)
(74) Vertreter: Weiss, Wolfgang, Dipl.-Chem. Dr.
(86) Internationale Anmeldenummer: EP9504308
(87) Internationale Veröffentlichungsnummer: WO9614338

(56) Entgegenhaltungen:
- EP-A- 0 292 810
- DE-A- 4 202 923
- PATENT ABSTRACTS OF JAPAN vol. 006 no. 073 (C-101) ,8.Mai 1982 & JP,A,57 009723 (MITSUBISHI CHEM IND LTD) 19.Januar 1982,
- THE JOURNAL OF RHEUMATOLOGY, Bd. 12, Nr. 3, Juni 1985 Seiten 427-431, RICHARD J. POWELL ET AL. 'AN IMPROVED ASSAY FOR IGG RHEUMATOID FACTOR: ITS VALUE IN THE DIAGNOSIS OF RHEUMATOID ARTHRITIS '

## Beschreibung

Die Erfindung betrifft die Verwendung einer aus mehreren verschiedenen Antikörpern oder/und Antikörperfragmenten bestehenden Zusammensetzung, als Reagenz zur Verringerung von durch Rheumafaktoren hervorgerufenen Störungen in einem immunchemischen Verfahren.

Der Säugetierorganismus enthält verschiedene Klassen von Antikörpern, die von B-Zellen des Immunsystems zur Bekämpfung von Antigenen gebildet werden. Die Antikörpermoleküle bestehen aus einem oder mehreren Sätzen von jeweils vier Polypeptidketten, zwei schweren Ketten und zwei leichten Ketten, die miteinander über Disulfidbrücken verbunden sind.

Antikörper werden im allgemeinen in die Klassen G, M, A, D und E eingeteilt. Diese fünf Immunglobulinklassen unterscheiden sich jeweils in der schweren Kette, die als γ-, µ-, α-, δ- bzw. ε-Kette bezeichnet wird. Darüber hinaus gibt es bei IgG, IgA und IgM auch Immunglobulinsubklassen.

Antikörper der Klasse IgG bilden in normalem menschlichem Serum 70 bis 75 % des Gesamtimmunglobulins (entsprechend 8 bis 16 mg/ml). Sie werden hauptsächlich als sekundäre Immunantwort des Organismus auf eine Infektion gebildet.

Antikörper der Klasse IgM bilden ca. 10 % des im humanen Serum vorhandenen Immunglobulins und besitzen eine pentamere Struktur. Diese Antikörper treten sehr früh nach einer Infektion auf, so daß ihre Bestimmung zur Früherkennung von Krankheiten bedeutsam ist.

Die Immunglobuline der Klasse IgA bilden ca. 15 bis 20 % des im humanen Serum vorhandenen Immunglobulins und stellen das wichtigste sekretorische Immunglobulin in Speichel, Milch und Sekreten des Urogenitalbereichs dar.

Antikörper der Klasse IgD finden sich auf der Membran zirkulierender B-Zellen und man nimmt an, daß sie bei Autoimmunkrankheiten eine Rolle spielen.

Antikörper der Klasse IgE kommen im Serum nur in sehr geringem Anteil vor, sie spielen aber eine wichtige Rolle bei einer Reihe allergischer Reaktionen, wie etwa Asthma und Heuschnupfen.

Die klassenspezifische Bestimmung von Immunglobulinen, d.h. die selektive Bestimmung von Antikörpern einer oder mehrerer ausgewählter Immunglobulinklassen oder -subklassen, die gegen ein bestimmtes Antigen gerichtet sind, in Gegenwart von Antikörpern anderer Immunglobulinklassen oder -subklassen, die gegen das gleiche Antigen gerichtet sind, ist von besonderer Bedeutung für den Nachweis bestimmter Erkrankungen, z.B. zur Früherkennung von Infektionen, zur Unterscheidung akuter von ausgeheilten Infektionen und zum Treffen präziser prognostischer Aussagen.

Verfahren zur klassenspezifischen Bestimmung von Immunglobulinen sind bekannt. Hierzu kann eine Immunkomponente, die für eine ausgewählte Antikörperklasse spezifisch ist, z.B. ein Antikörper gegen die µ-Kette von humanem IgM, an einen festen Träger gekoppelt werden und der antigenspezifische Immunglobulinanteil durch Reaktion mit einem direkt oder indirekt markierten Antigen nachgewiesen werden. Bei dieser Art von Verfahren und auch bei anderen immunologischen Testsystemen können Rheumafaktoren, d.h. Anti-IgG-Autoantikörper, die Bestimmung des Analyten stören. Dadurch kann es zu Ergebnisverfälschungen, insbesondere zu falsch-positiven Reaktionen kommen.

EP-A-0 163 312 offenbart ein immunologisches Testverfahren, bei dem Partikel, die eine durchschnittliche Größe von ≤ 0,2 µm aufweisen und mit einer Entstörsubstanz beladen sind, zur Hemmung einer unspezifischen Immunreaktion verwendet werden. Als Entstörsubstanzen werden Immunglobuline menschlichen oder tierischen Ursprungs genannt.

Henle et al. (Clin. Exp. Immunol. 36 (1979), 415 - 422) beschreiben Rheumafaktoren als Ursache von falsch-positiven Reaktionen in Tests auf Epstein-Barr-Virus spezifische Immunglobuline. Durch Verwendung von IgG-beschichteten Latexpartikeln konnten die Störungen in manchen Fällen beseitigt werden. Auch Ho et al. (J. Clin. Microbiol. 27 (1989), 952 - 958) beschreiben die Entstörung eines indirekten ELISA auf spezifische Anti-Epstein-Barr-Virus-Antikörper durch Zugabe mit von Human-IgG beschichteten Latexpartikeln.

Torfason und Diderholm (J. Med. Virol. 10 (1982), 157 - 170) offenbaren, daß Rheumafaktoren zu falschen Ergebnissen bei der Bestimmung von IgM-Immunglobulinen gegen Herpes Simplex Virus und Cytomegalovirus führen könnnen. Als Entstörungsreagenz wird eine Mischung von Protein A-Sepharose und mit humanem IgG gesättigter Protein-A-Sepharose genannt.

Eine Entstörung von Rheumafaktoren der Klassen IgM, IgA und IgG durch Zugabe von Dithiothreitol (DTT) wird von Espersen et al. (Scand J. Rheumatology 75 (1988), 40 -45) beschrieben.

EP-B-0 292 810 offenbart ein Verfahren zur Bestimmung von antigenspezifischen Antikörpern aus einer der Immunglobulinklassen M, A, D und E in einer Probeflüssigkeit, wobei zur Vermeidung von Störungen durch Antikörper der Klasse IgG ein Entstörungsreagenz zugesetzt wird, das aus Anti-Human-IgG, aggregiertem humanem oder tierischem IgG oder einem γ-Fc-Fragment ausgewählt ist. Das Entstörungsreagenz soll die Antigenbindung der in der Probe vorhandenen spezifischen IgG-Antikörper beseitigen und die Aktivität von Rheumafaktoren unterbinden.

DE-OS 42 02 923 beschreibt die Entstörung von Rheumafaktoren durch Zugabe nichtspezifischer Immunkomplexe. Diese Immunkomplexe enthalten Antikörper aus immunisierten Tieren, die gegen ein Antigen gerichtet sind, das nicht der Analyt ist.

Ein Verfahren zur Diagnostik akuter Infektionen durch IgM-Antikörperbestimmung ohne Rheumafaktor-Interferenz wird von R. Ziegelmaier et al. (Laboratoriumsblätter 33 (1983), 19 - 25) beschrieben. Als Entstörungsreagenzien werden aggregiertes γ-Globulin, IgG-beschichtete Polystyrolpartikel, Staphylokokken A-Protein und Antihuman-IgG-γ-Kette genannt.

Martins et al. (Clin. Diagnost. Laboratory Immunol. 2 (1995), 98 - 103) beschreiben eine Bewertung der Wirksamkeit von drei Prozeduren zur Entfernung von Immunglobulin-G beim klassenspezifischen Nachweis von IgM-Immunglobilinen. Es wurde festgestellt, daß Anti-Human-IgG-Antikörper eine höhere Wirksamkeit als rekombinantes Protein G besitzen.

Die Entstörungsreagenzien des Standes der Technik weisen jedoch einige Nachteile auf. So werden unlösliche Immunkomplexe erzeugt, die zur Trübung der Probe und gegebenenfalls zu Störungen bei der Messung führen können. Noch wichtiger ist, daß die offenbarten Reagenzien die Störungen durch Rheumafaktoren nicht vollständig beseitigen können, so daß selbst bei einer Sedimentation noch eine Verfälschung des Tests stattfinden kann. Bei Einsatz von Anti-Human-IgG-Antikörpern muß außerdem die Konzentration des Reagenz genau eingestellt werden, da bei einem Überschuß eine Auflösung des Präzipitats stattfindet.

Die deutsche Patentanmeldung DE-A-4 439 452 beschreibt eine aus mehreren verschiedenen Antikörpern oder/und Antikörperfragmenten bestehende Zusammensetzung, die als Entstörungsreagenz bei einem immunchemischen Verfahren zum klassenspezifischen Nachweis von Antikörpern aus einer oder mehreren der Immunglobulinklassen G, M, A, D und E geeignet ist. Diese Antikörper oder/und Antikörperfragmente sind spezifisch für den Fd-Abschnitt der schweren Kette von Immunglobulinen. Weiterhin wird die Verwendung der Zusammensetzung in einem Immunoassay zur selektiven klassenspezifischen Bestimmung von Antikörpern der Klassen IgG, IgM, IgA, IgD oder/und IgE vorgeschlagen, um die durch Antikörper anderer Klassen verursachten Störungen zu unterdrücken. Beispiele, in denen eine Entstörung von Rheumafaktoren gezeigt wird, sind nicht beschrieben.

R. Powell et al. (The Journal of Rheumatology 12, 3, 1985, 427 - 431) beschreiben einen verbesserten Test auf IgG Rheumafaktoren. Dabei wird die Bindung von IgG RF an immobilisiertes hu IgG Fc über gegen humanes IgG Fd gerichtetes radioaktiv markiertes F(ab')₂ quantifiziert.

Die der vorliegenden Erfindung zugrunde liegende Aufgabe bestand darin, ein Verfahren zur Entstörung von Rheumafaktoren bereitzustellen, bei dem die Nachteile des Standes der Technik mindestens weitgehend vermieden werden können.

Diese Aufgabe wird gelöst durch Verwendung einer Zusammensetzung, im wesentlichen bestehend aus mehreren verschiedenen Antikörpern oder/und Antikörperfragmenten, die spezifisch für den Fd-Abschnitt der schweren Kette von Immunglobulinen von einer oder mehreren der Klassen IgG, IgM, IgA, IgD und IgE ist und die Fähigkeit dieser Immunglobuline zur Antigenbindung zumindest weitgehend maskiert, als Reagenz zur Verringerung von durch Rheumafaktoren hervorgerufenen Störungen in einem immunchemischen Verfahren zur Bestimmung eines Analyten.

Die erfindungsgemäße Verwendung einer Anti-Fd-Antikörper- oder/und Antikörperfragmentzusammensetzung in einem immunchemischen Verfahren führt überraschenderweise zu einer weitgehenden oder vollständigen Entstörung von Rheumafaktoren. Besonders bevorzugt ist die Verwendung bei Testverfahren zur Bestimmung von IgM-Antikörpern, bei denen Rheumafaktoren, üblicherweise Rheumafaktoren der Klasse IgM, die unspezifisch gegen IgG-Antikörper, insbesondere humane IgG-Antikörper gerichtet sind, bei den Verfahren des Standes der Technik erhebliche Probleme bereitet haben.

Gegenüber den aus dem Stand der Technik bekannten Entstörungsverfahren wird eine Reihe von Vorteilen erzielt. So ist beim erfindungsgemäßen Verfahren keine Probenvorbehandlung erforderlich, d.h. die Anti-Fd-Zusammensetzung kann gleichzeitig mit anderen Testkomponenten zugesetzt werden. Weiterhin wirkt der Zusatz der Anti-Fd-Zusammensetzung keine Präzipitatbildung und eine dadurch hervorgerufene Störung des Tests. Auf diese Weise kann eine einfachere und damit sicherere Reagenzeinstellung sowie eine bessere Chargenkonstanz erreicht werden. Außerdem findet man eine deutliche Verringerung des Verschleppungsrisikos bei Messung in Analysenautomaten.

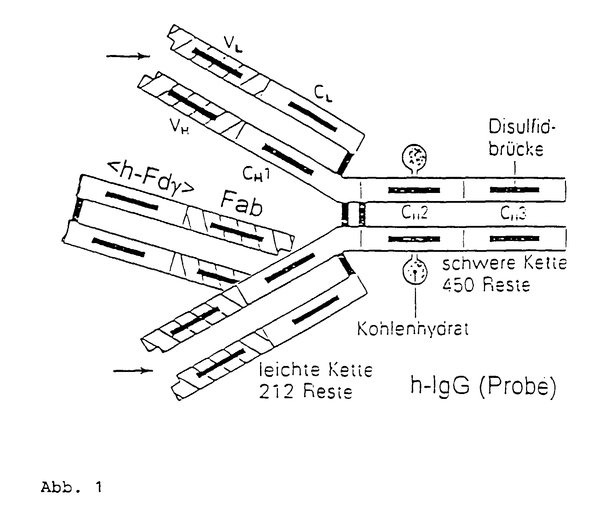

Die Bindung des Anti-Fd-Reagenz erfolgt an demjenigen Bereich der schweren Kette von Immunglobulinen, der zwischen der Antigenbindungsstelle und der Hinge-Region (im Bereich der V_{H} und der C_{H}¹-Domäne) liegt (vgl. Abb. 1). Vorzugsweise liegen die Bindungsstellen im konstanten Bereich der C_{H}¹-Domäne der Immunglobuline, also im C_{αH}¹-, C_{δH}¹-, C_{εH}¹-, C_{γH}¹- bzw. C_{µH}¹-Bereich (vgl. "Kurzes Lehrbuch der Immunologie", I.M. Roitt; Thieme Verlag, Stuttgart, New York (1987), Kapitel 5: "Antikörper: Struktur und Funktion", S. 49 ff.). Aminosäuresequenzen dieser Domänen sind z.B. bei Kabat et al., "Sequences of Proteins of Immunological Interest", 5th. ed. (1992), US Department of Health and Human Services, USA beschrieben.

Die durch Verwendung des Anti-Fd-Reagenz hervorgerufene Entstörung von Rheumafaktoren kann zur immunchemischen Bestimmung von beliebigen Analyten, z.B. Antigenen wie etwa Peptiden, Polypeptiden, Glykoproteinen, Hormonen, Mediatoren, Neurotransmittern, Metaboliten, Oberflächenstrukturen von Zellen oder Viren etc., aber auch zur spezifischen Bestimmung von Antikörpern eingesetzt werden. Bevorzugt ist die Bestimmung von Antikörpern gegen Pathogene wie etwa Viren, Bakterien oder anderen Mikroorganismen, oder von Autoimmun-Antikörpern. Vorzugsweise erfolgt die Bestimmung in Körperflüssigkeiten, insbesondere in humanen Körperflüssigkeiten, wie etwa Serum, Blut, Plasma, Speichel und Urin, in denen Rheumafaktoren enthalten sein können.

Die Zusammensetzung ist spezifisch für den Fd-Abschnitt der schweren Kette, d.h. sie ist im wesentlichen frei von Komponenten, die mit dem Fd-Abschnitt einer leichten Kette, d.h. einer κ- oder λ-Kette reagieren können. Die Kreuzreaktivität mit einer leichten Immunglobulinkette ist vorzugsweise maximal 10⁻¹ und besonders bevorzugt maximal 10⁻² bezüglich der Reaktivität mit der schweren Kette.

Die Antikörper- oder/und Antikörperfragmentzusammensetzung kann in einem 5-fachen molaren Überschuß bezüglich der zu maskierenden Rheumafaktoren deren Störungswirkung vorzugsweise um mindestens 50 %, besonders bevorzugt um mindestens 90 % und am meisten bevorzugt um mindestens 99 % verhindern.

Die erfindungsgemäß verwendete Zusammensetzung ist vorzugsweise spezifisch für eine erste Immunglobulinklasse ausgewählt aus den Klassen IgG, IgM, IgA, IgD und IgE und weist eine Kreuzreaktivität mit einer anderen Immunglobulinklasse von maximal 10⁻², besonders bevorzugt von maximal 10⁻³ bezüglich der Reaktivität gegenüber der ersten Immunglobulinklasse auf.

In einer bevorzugten Ausführungsform der Erfindung ist die Antikörper- oder/und Antikörperfragmentzusammensetzung spezifisch für den Fd-Abschnitt der schweren Kette von Immunglobulin G, insbesondere von humanem Immunglobulin G. Diese Zusammensetzung eignet sich insbesondere zur klassenspezifischen Bestimmung von Antikörpern aus einer oder mehreren der Klassen IgM, IgA, IgD und IgE in Anwesenheit von Rheumafaktoren, d.h. Anti-IgG-Antikörpern, und gegebenenfalls konkurrierenden Antikörpern der Klasse IgG. Eine derartige Zusammensetzung weist vorzugsweise eine Kreuzreaktivität mit einer anderen Immunglobulinklasse, z.B. IgM, von maximal 10⁻², besonders bevorzugt von maximal 10⁻³ bezüglich der Reaktivität gegenüber IgG auf.

Für einige Anwendungsformen kann es bevorzugt sein, daß die Zusammensetzung speziesspezifisch für humanes Immunglobulin ist und eine Kreuzreaktivität mit nicht-humanem Immunglobulin von maximal 10⁻², besonders bevorzugt von maximal 10⁻³ bezüglich der Reaktivität gegenüber humanem Immunglobulin aufweist.

Die erfindungsgemäß verwendete Zusammensetzung besteht aus Antikörpern oder/und Antikörperfragmenten. Vorzugsweise besteht die Zusammensetzung aus monovalenten Antikörperfragmenten, z.B. aus Fab-Fragmenten, die durch enzymatische Spaltung von Antikörpern mit Papain und anschließende Fraktionierung der Spaltprodukte erhältlich sind. Bei Verwendung einer aus monovalenten Antikörperfragmenten bestehenden Zusammensetzung wird eine Sedimentation der maskierten Antikörper vermieden.

Die erfindungsgemäße Zusammensetzung besteht aus mehreren verschiedenen Antikörpern oder/und Antikörperfragmenten, die spezifisch für jeweils unterschiedliche Bereiche des Fd-Abschnitts der schweren Immunglobulinkette sind. Vorzugsweise besteht die Zusammensetzung aus polyklonalen Antikörpern oder/und Antikörperfragmenten, sie kann jedoch auch aus einem Gemisch von mindestens zwei, vorzugsweise mindestens drei und besonders bevorzugt mindestens vier verschiedenen monoklonalen Antikörpern oder/und Antikörperfragmenten bestehen.

Eine erfindungsgemäß verwendete polyklonale Antikörper- oder/und Antikörperfragmentzusammensetzung ist erhältlich durch ein Verfahren, wobei man
(a) ein Versuchstier mit einem Immunogen, das den Fd-Abschnitt der schweren Kette von Immunglobulinen aus einer ersten Immunglobulinklasse enthält, immunisiert,
(b) ein polyklonales Antiserum aus dem Versuchstier gewinnt,
(c) das polyklonale Antiserum gegebenenfalls durch Spaltung in monovalente Antikörperfragmente überführt,
(d) das Antiserum bzw. die monovalente Antikörperfragmente einem oder mehreren Immunsorptionsschritten unterzieht, die eine Selektion auf Antikörper bzw. Antikörperfragmente erlauben, die spezifisch für den Fd-Abschnitt der schweren Kette der ersten Immunglobulinklasse sind, und
(e) eine Antikörper- bzw. Antikörperfragmentzusammensetzung mit einer Spezifität für den Fd-Abschnitt der ersten Immunglobulinklasse gewinnt.

Die Verwendung eines geeigneten Immunogens in Kombination mit der Durchführung geeigneter Immunsorptionsschritte ermöglicht die Gewinnung einer Antikörper- bzw. Antikörperfragmentzusammensetzung, die für den Fd-Abschnitt der schweren Kette einer oder mehrerer ausgewählter Immunglobulinklassen spezifisch ist und vorzugsweise im wesentlichen keine Kreuzreaktivität mit anderen Immunglobulinklassen oder speziesfremden Immunglobulinen der ausgewählten Klassen zeigt. Als Immunogene können z.B. vollständige Antikörper, Antikörperfragmente, z.B. durch enzymatische Spaltung erzeugte Antikörperfragmente wie etwa Fab, Fab' oder F(ab)'₂ oder rekombinante Antikörperfragmente, Peptidepitope oder Gemische davon verwendet werden. Die Verwendung von Immunogenen, die frei von Antikörper-Fc-Bestandteilen sind, ist bevorzugt. Auf diese Weise kann im Versuchstier, z.B. einem Schaf, einem Kaninchen oder einer Maus, die Entstehung von Anti-Fc-Antikörpern weitgehend vermieden werden.

Die Immunsorptionsschritte des erfindungsgemäßen Verfahrens umfassen vorzugsweise (i) mindestens eine positive Immunsorption gegen ein Antigen, das den Fd-Abschnitt der schweren Kette der ersten Immunglobulinklasse enthält, und die Gewinnung von bindenden Komponenten der Zusammensetzung, die spezifisch den Fd-Abschnitt der schweren Kette der ersten Immunglobulinklasse erkennen. Bei Verwendung eines Immunogens, das den Fd-Abschnitt der schweren Kette von Immunglobulin G enthält, z.B. eines Fab-Fragments, werden das Antiserum bzw. die Antikörperfragmente einer positiven Immunsorption gegen Immunglobulin G oder Fragmente davon, die den Fd-Abschnitt enthalten, unterzogen. Die Verwendung von Fc-freien Antigenen, z.B. Fab-Fragmenten, bei der positiven Immunsorption ist empfehlenswert, wenn als Immunogen ein komplettes Immunglobulin verwendet wurde.

Die Immunsorptionsschritte umfassen vorzugsweise weiterhin (ii) mindestens eine negative Immunsorption gegen Antigene, die aus Immunglobulinklassen, die von der ersten Immunglobulinklasse verschieden sind, oder Bestandteilen davon ausgewählt sind, und die Gewinnung von nicht-bindenden Komponenten der Zusammensetzung. Durch diese negative Immunsorption wird die Kreuzreaktivität der Zusammensetzung gegen den Fd-Abschnitt der leichten Kette und gegen den Fd-Abschnitt der schweren Kette anderer Immunglobulinklassen weitgehend vermindert. Zur Herstellung einer für den Fd-Abschnitt von Immunglobulin G spezifischen Zusammensetzung kann die negative Immunsorption gegen IgM, IgD, IgE oder/und IgA oder Bestandteile davon durchgeführt werden.

Gegebenenfalls können die Immunsorptionsschritte weiterhin (iii) mindestens eine negative Immunsorption gegen Antigene, die aus der ersten Immunglobulinklasse einer Spezies, die von der Spezies der Immunglobuline im positiven Immunsorptionsschritt (i) verschieden ist, oder Bestandteilen davon ausgewählt sind, und die Gewinnung von nicht-bindenden Komponenten der Zusammensetzung umfassen. Die Durchführung dieses Schrittes ist bei einer späteren Verwendung der Zusammensetzung in einem Immunoassay bevorzugt, bei dem ein Detektionsantikörper der gleichen Immunglobulinklasse, aber aus einer anderen Spezies, verwendet wird. Vorzugsweise wird die positive Immunsorption (i) mit humanen Immunglobulinen und die negative Immunsorption (iii) mit Immunglobulinen einer nicht humanen Spezies, z.B. Maus, durchgeführt. Bei Herstellung einer für den Fd-Abschnitt von humanem Immunglobulin G spezifischen Zusammensetzung erfolgt der negative Immunsorptionsschritt (iii) gegen Immunglobulin G einer nicht-humanen Spezies.

Die Durchführung der Immunsorptionsschritte erfolgt vorzugsweise durch Adsorptionschromatographie an Säulen, die die jeweils verwendeten Antigene in immobilisierter Form enthalten. Prozeduren zur Gewinnung gereinigter Antigenpräparationen und zur Immobilisierung von Antikörpern bzw. Antikörperfragmenten an ein Trägermaterial sind dem Fachmann wohlbekannt (siehe z.B. EP-A-0 394 819, insbesondere Beispiel 7).

Die Antikörper- oder/und Antikörperfragmentzusammensetzung kann vorzugsweise in einem Immunoassay zur selektiven klassenspezifischen Bestimmung von Antikörpern der Klassen IgM, IgA, IgD oder/und IgE verwendet werden, um die durch Rheumafaktoren verursachten Störungen zu unterdrücken. Hierzu wird beispielsweise einer zu vermessenden Humanserum- oder Plasmaprobe, in der ein gegen ein bestimmtes, z.B. virales oder bakterielles Antigen gerichteter Antikörper einer bestimmten Immunglobulinklasse (z.B. IgM) klassenspezifisch quantifiziert werden soll, eine erfindungsgemäße IgG-spezifische Zusammensetzung, vorzugsweise nach einem Vorverdünnungsschritt zugesetzt, so daß eine Entstörung der in der Probe vorhandenen Rheumafaktoren erfolgt. Beim anschließenden immunologischen Nachweis werden nur noch die spezifischen IgM-Antikörper, nicht jedoch unspezifische Anti-IgG-Rheumafaktoren in der Probe erfaßt. Auf diese Weise ist eine differentielle Quantifizierung des spezifischen Gehalts einer bestimmten Antikörperklasse ohne Störung durch anwesende Rheumafaktoren möglich. Die Verwendung der erfindungsgemäßen Zusammensetzung erlaubt eine Einschritt-Testführung ohne Waschvorgang.

Ein Gegenstand der vorliegenden Erfindung ist somit auch ein Verfahren zur immunchemischen Bestimmung eines Analyten, z.B. von spezifischen Antikörpern aus einer oder mehreren ausgewählten Immunglobulinklassen, in einer Probeflüssigkeit, die auch Rheumafaktoren enthält, welches dadurch gekennzeichnet ist, daß man die Bestimmung in Gegenwart einer wie vorstehend definierten Anti-Fd-Zusammensetzung durchführt. Die Zusammensetzung wird vorzugsweise in einer Menge zugesetzt, die einem mindestens 5-fachen molaren Überschuß der aktiven Komponenten gegenüber den in der Probeflüssigkeit vorhandenen Rheumafaktoren entspricht. Die Zusammensetzung wird vorzugsweise in einem 10- bis 1000-fachen molaren Überschuß zugesetzt. Gegebenenfalls wird eine Vorinkubation mit einer Dauer von vorzugsweise 5 bis 60 min und besonders bevorzugt 10 bis 30 min durchgeführt.

Besonders bevorzugt wird eine Bestimmung spezifischer Antikörper nach dem Prinzip des heterogenen Immunoassay in Gegenwart einer reaktiven Festphase und zwei mit den nachzuweisenden Antikörpern bindefähigen Rezeptoren R₁ und R₂ durchgeführt, wobei R₁ an die Festphase gebunden oder zur Bindung an die Festphase fähig ist und R₂ direkt oder indirekt markiert ist. Die nachzuweisenden Antikörper können dann gegebenenfalls nach Trennung von Festphase und Inkubationsflüssigkeit durch Messung der Markierung in der Festphase oder/und in der Inkubationsflüssigkeit bestimmt werden.

Als festphasenseitigen Rezeptor R₁ kann man ein Konjugat aus einem mit den nachzuweisenden Antikörpern spezifisch reagierenden Antigen und einer Festphasenbindungsgruppe verwenden. In diesem Falle kann der Rezeptor R₂ entweder ein mit den nachzuweisenden Antikörpern spezifisch reagierendes Antigen oder ein die ausgewählte Antikörperklasse erkennender Antikörper, z.B. ein Anti-IgM-Antikörper bzw. ein entsprechendes Antikörperfragment, sein. Der Rezeptor R₂ kann direkt oder indirekt markiert sein, d.h. er kann mit einer Markierungsgruppe gekoppelt sein oder er kann an einen weiteren Rezeptor binden, der seinerseits eine Markierungsgruppe trägt.

Als festphasenseitigen Rezeptor R₁ kann man auch ein Konjugat aus einem die ausgewählte Antikörperklasse erkennenden Antikörper bzw. einem entsprechenden Antikörperfragment und einer Festphasenbindungsgruppe verwenden. In diesem Falle verwendet man als markierten Rezeptor R₂ vorzugsweise ein mit den nachzuweisenden Antikörpern spezifisch reagierendes Antigen.

Vorzugsweise verwendet man eine mit Streptavidin oder Avidin beschichtete Festphase und einen biotinylierten Rezeptor R₁, der an diese Festphase binden kann. Als Markierung kann man chromophore Stoffe, radioaktive Isotope, Enzyme, NMR-Markierungen oder alle anderen aus dem Stand der Technik bekannten Markierungen verwenden. Vorzugsweise verwendet man lumineszierende Metallkomplexe, bei denen die Messung der Markierung durch Elektrochemilumineszenz erfolgen kann. Beispiele für lumineszierende Metallkomplexe sowie Verfahren und Vorrichtungen zur Elektrochemilumineszenzmessung sind in EP-A-0 199 804, EP-A-0 580 979, WO87/06706, WO90/05301, WO90/11511 und W092/14138 beschrieben. Eine weitere bevorzugte Markierung sind Enzyme (z.B. Peroxidase, alkalische Phosphatase oder β-Galactosidase), bei denen die Messung der Markierung durch Nachweis der jeweiligen enzymatischen Reaktion erfolgen kann.

Weiterhin wird die Erfindung durch die folgenden Beispiele und Abbildungen erläutert.

Es zeigen:
- Abb. 1: die Bindung eines gegen den Fd-Abschnitt der schweren Kette gerichteten Fab-Fragments (<h-Fdγ>Fab) an humanes IgG (h-IgG),
- Abb. 2: das Testprinzip des in Beispiel 3 durchgeführten klassenspezifischen Nachweises von Anti-HBc-IgM durch Elektrochemilumineszenz und
- Abb. 3: ein weiteres Testprinzip für einen klassenspezifischen Nachweis von Anti-HBc-IgM durch Elektrochemilumineszenz.

### Beispiel 1

### Herstellung eines erfindungsgemäßen Reagenz

### 1.1 Herstellung des Immunogens

Aus Humanserum wird nach üblichen Standardverfahren die Immunglobulin G-Fraktion aufgereinigt. Diese Aufreinigung kann beispielsweise durch Aerosil-Delipidierung, Fällung mit Ammoniumsulfat, Chromatographie an DEAE-Sepharose FF und gegebenenfalls Immunsorption an immobilisierten IgG-spezifischen polyklonalen Antikörpern erfolgen.

Die so gereinigte Immunglobulinfraktion wird mit Papain in Fab- und Fc-Fragmente gespalten. Die Spaltung wird vorzugsweise bei pH 7 mit einer IgG-Konzentration von 10 mg/ml, 40 mU/ml Papain 7 und 10 mmol/l Cystein bei 37°C durchgeführt. Die Fab-Anteile werden durch DEAE-Sepharose FF-Chromatographie und Gelchromatographie (z.B. Sephacryl TSK S200 und S300) von den Fc-Anteilen gereinigt. Die Fab-Fragmente werden zur Steigerung der Immunreaktion vorzugsweise an ein Trägerprotein, z.B. Maleimidaktiviertes Keyhole Limpet-Hämocyanin (Boehringer Mannheim, Biochemica-Katalog, Best.Nr. 1376438) gekoppelt. Hierzu werden zunächst die Fab-Fragmente mit N-Succinimidyl-S-acetylthiopropionat (SATP) in einem molaren Verhältnis von 1:6 aktiviert. Die Kopplung des Trägerproteins mit dem aktivierten Fab-Fragment erfolgt in einem molaren Verhältnis von 1:1.

Auf entsprechende Weise können auch Fab-Fragmente anderer Immunglobulinklassen (z.B. Fabµ) hergestellt werden.

### 1.2 Immunisierung von Schafen

Mit dem in Beispiel 1 hergestellten Immunogen werden nach üblichen Verfahren Schafe immunisiert. Diese bilden in einer Immunreaktion polyklonale Antikörper gegen humanes Fab, die sowohl gegen den Anteil der leichten Kette als auch gegen den Anteil der schweren Kette in humanem Fab gerichtet sind.

Den immunisierten Schafen wird Rohserum entnommen und daraus die IgG-Fraktion nach dem bereits beschriebenen Verfahren gewonnen.

### 1.3 Herstellung eines Fd-spezifischen Reagenz

Die Schaf-Immunglobulin-G-Fraktion wird mit Papain proteolytisch gespalten. Die Fab-Fragmente werden von den übrigen Spaltungsprodukten mittels DEAE-Anionenaustauscherchromatographie abgetrennt.

Aus der Gesamt-Schaf-Fab-Fraktion werden die gegen den Fd-Abschnitt der schweren Kette gerichteten Anteile durch mehrere Immunsorptionsschritte aufgereinigt.

Durch gegebenenfalls mehrfache Passage eines Immunadsorbers, auf dem humanes IgM immobilisiert ist, können die spezifisch gegen die leichte Kette (κ oder λ) des humanen IgG gerichteten Anteile entfernt werden, weil der konstante Bereich der leichten Kette von IgG und IgM homolog ist. Während die gegen die leichte Kette gerichteten Anteile auf der Säule adsorbiert werden, befinden sich die gegen den Fd-Abschnitt der schweren Kette gerichteten Anteile im Säulendurchfluß. Als Säule wird eine mit polyklonalem IgM gekoppelte Spherosilsäule verwendet. Der Auftragspuffer ist z.B. PBS/Azid (50 mmol/l K-Phosphat pH 7,5; 150 mmol/1 NaCl; 0,1 % Natriumazid).

Die nicht bindenden Anteile aus der IgM-Säule werden vereinigt und einer gegebenenfalls mehrfachen Passage eines weiteren Immunadsorbers unterworfen, auf dem humanes IgG immobilisiert ist. Hier können die spezifischen Anteile an Anti-Fd-Schaf-Fab von unspezifischen Schaf-Fab-Anteilen getrennt werden. Das Fd-spezifische Schaf-Fab bindet an diese Säule und kann mit einem Elutionspuffer, z.B. 1 mol/l Propionsäure, eluiert werden. Die übrigen Komponenten binden nicht und werden durch Waschen der Säule mit einem geeigneten Puffer (z.B. PBS/Azid) entfernt.

Weiterhin können störende Kreuzreaktivitäten gegen die üblicherweise in immunologischen Tests als Bindepartner verwendeten Maus-Antikörper durch zusätzliche Passage eines Immunadsorbers mit immobilisiertem Maus-IgG entfernt werden. Die gewünschten Anteile der Präparation sind im Säulendurchfluß.

Die Bindung eines für humanes Fdγ spezifischen Fab-Fragments (<h-Fdγ>Fab) an humanes IgG (h-IgG) ist schematisch in Abb. 1 gezeigt. Die Antigenbindungsstellen des h-IgG sind jeweils mit Pfeilen gekennzeichnet.

### Beispiel 2

### Überprüfung der Reinheit des Reagenz

Mikrotiterplatten (MTP) der Fa. Nunc wurden mit Schaf-Fabspezifischem Kaninchen-IgG als Festphasen- oder Fängerantikörper beschichtet (10 µg/ml Kaninchen-IgG, Na-Carbonatpuffer pH 9,6, Nachbeschichtung mit 1 % Rinderserumalbumin in PBS-Puffer). Das zu analysierende Reagenz wird als Probe mit jeweils steigenden Konzentrationen (z.B. 0 bis 10 µg/ml) einer Präparation aus gereinigten humanen Fabγ-Fragmenten bzw. humanen Fabµ-Fragmenten vorinkubiert und anschließend in die beschichteten MTP überführt (Probenvolumen 200 µl, Inkubation 2 h bei Raumtemperatur, Puffer PBS mit 1 % Rinderserumalbumin).

Die für humanes Fdy spezifischen Schaf-Fab-Komponenten der Probe werden ebenso wie gegebenenfalls vorhandene Verunreinigungen, die gegen die leichte Immunglobulinkette gerichtet sind und bei der Aufreinigung möglicherweise nicht vollständig abgetrennt wurden, an die Festphase gebunden. Nicht gebundene Anteile werden durch dreimaliges Waschen mit PBS-Puffer entfernt.

Als Nachweisreagenz wird eine an humanes Fabγ gekoppelte Meerrettich-Peroxidase eingesetzt (200 µl, 50 mU/ml). Nach dreimaligem Waschen mit PBS-Puffer wird die enzymatische Aktivität des gebundenen Peroxidasekonjugats durch einstündige Inkubation bei Raumtemperatur mit dem Substrat ABTS® entwickkelt und anschließend photometrisch bei 405 nm vermessen. Von den zuvor an die Festphase gebundenen Schaf-Fab-Komponenten der Probe werden vom Konjugat-Enzymmarker nur die gegen humanes Fab gerichteten Anteile erkannt. Dadurch wird in der Kontrolle ohne Fabµ- und Fabγ-Zusatz ein Positivsignal von 1000 bis 3000 mE erzeugt. Dieses entspricht der Gesamtmenge des in einer zu untersuchenden Präparation vorhandenen, gegen humanes Fab gerichteten Schaf-Fab. In den Proben, denen steigende Menge an humanem Fabγ zugesetzt wurden, kommt es durch Maskierung des Fdγ-spezifischen Schaf-Fab zu einer zunehmenden Verdrängung des Konjugats und somit zu einer gegen 0- bzw. gegen einen Leerwert strebenden Kurve des Meßsignals. In den Proben, denen steigende Mengen an humanem Fabµ beigemischt wurden, können dagegen nur die gegen die leichte Kette gerichteten Schaf-Fab-Anteile maskiert werden, so daß es hier nur zu einem Signalabfall kommt, wenn solche unerwünschten Komponenten noch in der Schaf-Fab-Präparation verhanden sind. Die Differenz der beiden Verdrängungskurven würde in letzterem Fall dem tatsächlichen Anteil an Fdγ-spezifischen Schaf-Fab in der Präparation entsprechen.

Die Messung der Probe ohne zugesetztes humanes Fabµ bzw. Fabγ ergibt eine Extinktion von 1600 mE. Die Probe + Fabµ ergibt eine Extinktion von 1500 mE. Die Probe + Fabγ ergibt eine Extinktion von 150 mE.

### Beispiel 3

### Nachweis von Anti-HBc-IgM durch Elektrochemilumineszenz in Gegenwart von Rheumafaktoren

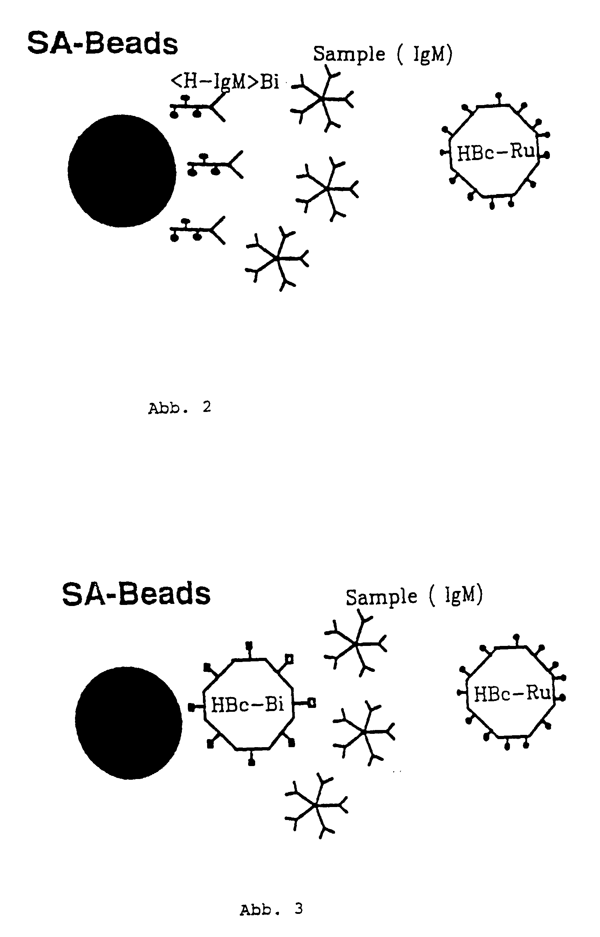

Zur Messung wurde ein Apparat verwendet, wie er in WO90/05302 beschrieben ist. Das Testprinzip ist in Abb. 2 dargestellt und beruht auf der Verwendung eines biotinylierten, gegen humanes IgM spezifischen Antikörpers (<H-IGM>Bi) und eines direkt mit einem Rutheniumkomplex markierten HBc-Antigens (HBc-Ru). Alternativ kann auch ein indirekt (durch Bindung eines ruthenylierten Anti-HBc-Antikörpers) markiertes HBc-Antigens eingesetzt werden. Der Rutheniumkomplex und die zur Kopplung an das HBc-Antigen bzw. an den Antikörper verwendeten Techniken sind in EP-A-0 199 804 beschrieben.

Ein weiteres Testprinzip ist in Abb.3 gezeigt. Es beruht auf der Verwendung eines biotinylierten HBc-Antigens (HBc-Bi) und eines Ruthenium-markierten HBc-Antigens (HBc-Ru).

### Die Durchführung war wie folgt:

90 µl einer Lösung von unspezifischem humanem IgM (11 µg/ml) mit bzw. ohne aggregiertem IgG (0,5 mg/ml) bzw. Anti-Fdγ-Reagenz (2 mg/ml) wurden mit 10 µl von 1:100 vorverdünnten Proben bzw. Standards versetzt und 10 min bei 37°C inkubiert.

Anschließend wurden 100 µl einer Lösung, die Ruthenium-markiertes HBc-Antigen (0,5 µg/ml) und einen biotinylierten Anti-human-IgM-Antikörper (2 µg/ml) enthielt, sowie 40 µl Streptavidin beschichtete Magnetpartikel (Fa. Dynal, 720 µg/ml) zugesetzt, das Gemisch danach weitere 10 min bei 37°C inkubiert, anschließend mit Assaypuffer (200 mmol/1 Phosphat, pH 6,8, Polydocanol 0,1 %, Oxaban A 0,1 %, Tripropylamin 160 mmol/l) in die auf 28°C temperierte Meßzelle überführt und dort vermessen.

Die Anti-HBc-IgM-Konzentration in den Proben wurde anschließend anhand einer Eichkurve bestimmt. Die Ergebnisse auf Konzentrationsbasis sind in der Tabelle 1 dargestellt.

Es ist zu erkennen, daß ohne Zusatz von Entstörungsreagenz bzw. nur bei Zusatz von aggregiertem IgG scheinbar vier Proben (1, 3, 4 und 5) eine positive Reaktion zeigen. In Gegenwart des Anti-Fd-Reagenz zeigt nur die Probe Nr. 1 eine positive Reaktion. Das positive Signal bei den Proben 3 - 5 in Abwesenheit von Anti-Fd-Reagenz ist somit ein falsch-positives Signal und nur auf die Gegenwart hoher Konzentrationen an Rheumafaktoren (RF) zurückzuführen.

Wie aus der Tabelle ersichtlich ist, führt die Zugabe von Anti-Fd-Reagenz (<Fdy>) im Gegensatz zur Zugabe von aggregiertem IgG gemäß Stand der Technik zu einer vollständigen Entstörung der Rheumaseren. Dieses Ergebnis zeigt, daß das Anti-Fd-Reagenz eine weit bessere Entstörungswirkung als das aus dem Stand der Technik bekannte unspezifische IgG besitzt.

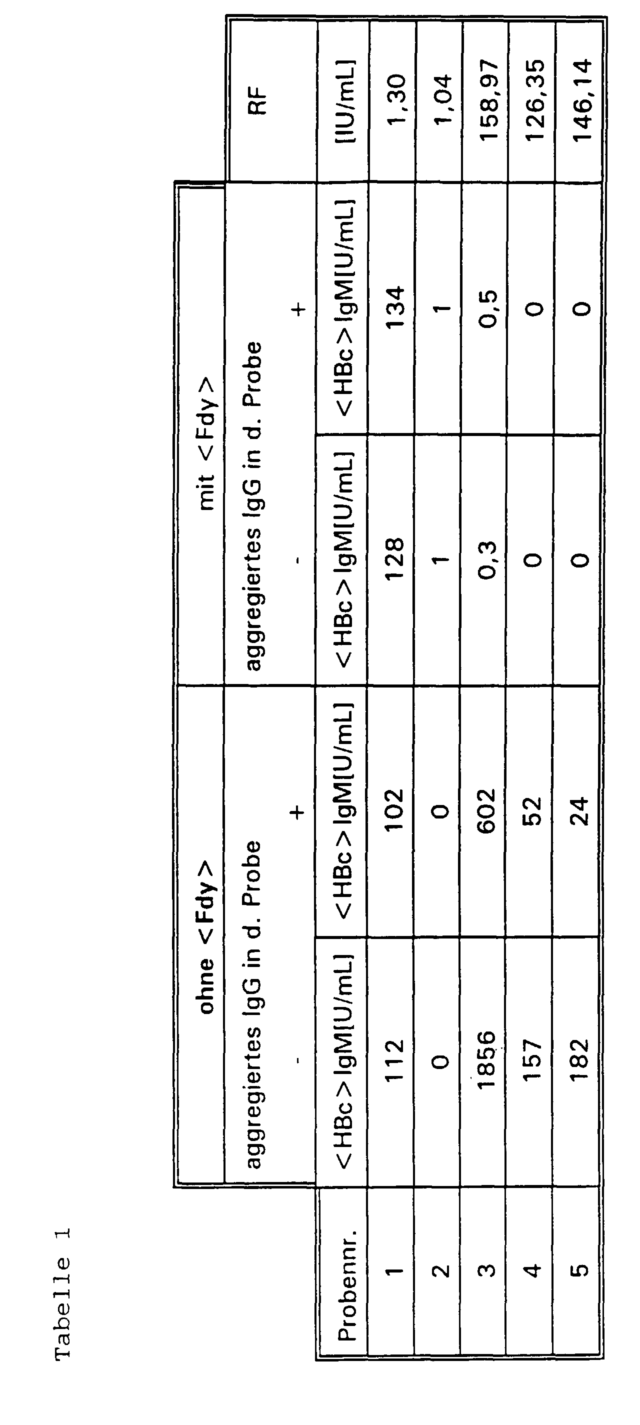

## Patentansprüche

1. Verwendung einer Zusammensetzung, im wesentlichen bestehend aus mehreren verschiedenen Antikörpern oder/und Antikörperfragmenten, die spezifisch für den Fd-Abschnitt der schweren Kette von Immunglobulinen aus einer oder mehreren der Klassen IgG, IgM, IgA, IgD und IgE ist und die Fähigkeit dieser Immunglobuline zur Antigenbindung zumindest weitgehend maskiert, als Reagenz zur Verringerung von durch Rheumafaktoren hervorgerufenen Störungen in einem immunchemischen Verfahren zur Bestimmung eines Analyten.

2. Verwendung nach Anspruch 1,
**dadurch gekennzeichnet**,
daß die Zusammensetzung spezifisch für den Fd-Abschnitt der schweren Kette von Immunglobulin G ist.

3. Verwendung nach Anspruch 1 oder 2,
**dadurch gekennzeichnet**,
daß die Zusammensetzung in einem 5-fachen molaren Überschuß bezüglich der Rheumafaktoren deren Störungswirkung um mindestens 50 % verhindert.

4. Verwendung nach einem der vorhergehenden Ansprüche,
**dadurch gekennzeichnet**,
daß die Zusammensetzung spezifisch für IgG ist und eine Kreuzreaktivität mit einer anderen Immunglobulinklasse von maximal 10⁻² bezüglich der Reaktivität gegenüber IgG aufweist.

5. Verwendung nach einem der vorhergehenden Ansprüche,
**dadurch gekennzeichnet**,
daß die Zusammensetzung spezifisch für humanes Immunglobulin ist und eine Kreuzreaktivität mit nicht-humanem Immunglobulin von maximal 10⁻² bezüglich der Reaktivität gegenüber humanem Immunglobulin aufweist.

6. Verwendung nach einem der Ansprüche 1-5 in einem Immunoassay zur selektiven klassenspezifischen Bestimmung von Antikörpern der Klassen IgM, IgA, IgD oder/und IgE in einer Rheumafaktoren enthaltenden Probe.

7. Verwendung nach einem der Ansprüche 1 - 6 in einem Einschritt-Immunoassay.

8. Verfahren zur immunchemischen Bestimmung eines Analyten in einer Probeflüssigkeit, die auch Rheumafaktoren enthält,
**dadurch gekennzeichnet,**
daß man die Bestimmung in Gegenwart einer Zusammensetzung durchführt, die im wesentlichen aus mehreren verschiedenen Antikörpern oder/und Antikörperfragmenten besteht, die spezifisch für den Fd-Abschnitt der schweren Kette von Immunglobulinen aus einer oder mehreren der Klassen IgG, IgM, IgA, IgD und IgE ist und die Fähigkeit dieser Immunglobuline zur Antigenbindung zumindest weitgehend maskiert.

9. Verfahren nach Anspruch 8,
**dadurch gekennzeichnet**,
daß man die Zusammensetzung in einer Menge zusetzt, die einem mindestens 5-fachen molaren Überschuß der aktiven Komponenten gegenüber den in der Probeflüssigkeit vorhandenen Rheumafaktoren entspricht.

10. Verfahren nach einem der Ansprüche 8 bis 9,
**dadurch gekennzeichnet**,
daß die Bestimmung nach dem Prinzip des heterogenen Immunoassay in Gegenwart einer reaktiven Festphase und zwei mit den nachzuweisenden Analyten bindefähigen Rezeptoren R₁ und R₂ erfolgt, wobei R₁ an die Festphase gebunden oder zur Bindung an die Festphase fähig ist und R₂ direkt oder indirekt markiert ist und die nachzuweisenden Analyten durch Messung der Markierung in der Festphase oder/und in der Inkubationsflüssigkeit bestimmt werden.

## Claims

1. Use of a composition which is essentially composed of several different antibodies or/and antibody fragments which is specific for the Fd section of the heavy chain of immunoglobulins from one or several of the classes IgG, IgM, IgA, IgD and IgE and which substantially masks the ability of these immunoglobulins to bind antigens, as a reagent for reducing interferences caused by rheumatoid factors in an immunochemical method for the determination of an analyte.

2. Use as claimed in claim 1,
**wherein**
the composition is specific for the Fd section of the heavy chain of immunoglobulin G.

3. Use as claimed in claim 1 or 2,
**wherein**
the composition in a 5-fold molar excess relative to the rheumatoid factors prevents their interfering effect by at least 50 %.

4. Use as claimed in one of the previous claims,
**wherein**
the composition is specific for IgG and has a maximum cross-reactivity with another immunoglobulin class of 10⁻² relative to the reactivity towards IgG.

5. Use as claimed in one of the previous claims,
**wherein**
the composition is specific for human immunoglobulin and has a maximum cross-reactivity with non-human immunoglobulin of 10⁻² relative to the reactivity towards human immunoglobulin.

6. Use as claimed in one of the claims 1-5 in an immunoassay for the selective class-specific determination of antibodies of the classes IgM, IgA, IgD or/and IgE in a sample containing rheumatoid factors.

7. Use as claimed in one of the claims 1 - 6 in a one-step immunoassay.

8. Method for the immunochemical determination of an analyte in a sample liquid which also contains rheumatoid factors,
**wherein**
the determination is carried out in the presence of a composition which is essentially composed of several different antibodies or/and antibody fragments which is specific for the Fd section of the heavy chain of immunoglobulins of one or several of the classes IgG, IgM, IgA, IgD and IgE and at least masks the ability of these immunoglobulins to bind antigen.

9. Method as claimed in claim 8,
**wherein**
the composition is added in an amount which corresponds to an at least 5-fold molar excess of the active component relative to the rheumatoid factors present in the sample liquid.

10. Method as claimed in one of the claims 8 to 9,
**wherein**
the determination is carried out according to the principle of a heterogeneous immunoassay in the presence of a reactive solid phase and two receptors R₁ and R₂ capable of binding to the analyte to be detected, in which R₁ is bound to the solid phase or is capable of binding to the solid phase and R₂ is directly or indirectly labelled and the analytes to be detected are determined by measuring the label in the solid phase or/and in the incubation liquid.

## Revendications

1. Utilisation d'une composition, constituée essentiellement de plusieurs anticorps et/ou de fragments d'anticorps différents, qui est spécifique du fragment Fd de la chaîne lourde d'immunoglobulines d'une ou plusieurs des classes IgG, IgM, IgA, IgD et IgE, et qui bloque au moins dans une large mesure la capacité de ces immunoglobulines à lier des antigènes, comme réactif pour la réduction des perturbations dues aux facteurs rhumatoïdes dans un procédé immunochimique de dosage d'un analyte.

2. Utilisation selon la revendication 1, caractérisée en ce que la composition est spécifique du fragment Fd de la chaîne lourde d'une immunoglobuline G.

3. Utilisation selon la revendication 1 ou 2, caractérisée en ce que la composition, en un excès molaire de 5 fois par rapport aux facteurs rhumatoïdes, inhibe à au moins 50 % leur effet perturbateur.

4. Utilisation selon l'une des revendications précédentes, caractérisée en ce que la composition est spécifique des IgG et présente vis-à-vis d'une autre classe d'immunoglobulines une réactivité croisée d'au plus 10⁻² par rapport à la réactivité vis-à-vis des IgG.

5. Utilisation selon l'une des revendications précédentes, caractérisée en ce que la composition est spécifique d'une immunoglobuline humaine et présente vis-à-vis d'une immunoglobuline non humaine une réactivité croisée d'au plus 10⁻² par rapport à la réactivité vis-à-vis d'une immunoglobuline humaine.

6. Utilisation selon l'une des revendications 1 à 5 dans une analyse immunologique pour le dosage sélectif, spécifique d'une classe, d'anticorps des classes IgM, IgA, IgD et/ou IgE dans un échantillon contenant des facteurs rhumatoïdes.

7. Utilisation selon l'une des revendications 1 à 6 dans une analyse immunologique en une étape.

8. Procédé de dosage immunochimique d'un analyte dans un échantillon liquide contenant aussi des facteurs rhumatoïdes, caractérisé en ce que l'on effectue le dosage en présence d'une composition constituée essentiellement de plusieurs anticorps et/ou fragments d'anticorps différents, qui est spécifique du fragment Fd de la chaîne lourde d'immunoglobulines d'une ou plusieurs des classes IgG, IgM, IgA, IgD et IgE, et qui bloque au moins dans une large mesure la capacité de ces immunoglobulines à lier des antigènes.

9. Procédé selon la revendication 8, caractérisé en ce que l'on ajoute la composition en une quantité qui correspond à un excès molaire d'au moins 5 fois des constituants actifs par rapport aux facteurs rhumatoïdes présents dans l'échantillon liquide.

10. Procédé selon l'une des revendications 8 à 9, caractérisé en ce que l'on effectue le dosage selon le principe de l'analyse immunologique hétérogène en présence d'une phase solide réactive et de deux récepteurs R₁ et R₂ capables de se lier aux analytes à déceler, R₁ étant lié à la phase solide ou capable de se lier à la phase solide et R₂ étant marqué directement ou indirectement, et on dose les analytes à déceler en mesurant le marqueur dans la phase solide et/ou dans le liquide d'incubation.
